# EUROPEAN PATENT SPECIFICATION

(11) **EP 0 589 928 B1**
(45) Date of publication and mention of the grant of the patent: **09.06.2004**
(21) Application number: 92911435.3
(22) Date of filing: 12.06.1992
(51) Int. Cl.: C12N 9/24, G01N 33/53, A23L 3/36, A23G 9/00

(54) **COLD TOLERANCES IN PLANTS**
KÄLTETOLERANZ BEI PFLANZEN
RESISTANCE DES VEGETAUX AU FROID

(30) Priority: 13.06.1991 GB 91127746; 13.12.1991 GB 91264853
(43) Date of publication of application: 06.04.1994
(73) Proprietor: Microstar Biotech Inc., Hamilton, Ontario L9H 7H9 (CA)
(72) Inventor: GRIFFITH, Marilyn, Waterloo, Ontario N2L 1E3 (CA)
(74) Representative: Smart, Peter John
(86) International application number: PCT/CA1992/000255
(87) International publication number: WO 1992/022581

(56) References cited:
- WO-A-90/13571
- PLANT PHYSIOLOGY. vol. 99, no. 1, May 1992, SUPPLEMENT. ROCKVILLE, MD, USA. page 126; GRIFFITH, M., ET AL.: 'Antifreeze protein produced by winter rye leaves'
- Biological Abstracts vol.92,1991,ref.57717
- Biological Abstracts BR39:89008
- Biological Abstracts vol76,1983,ref.37192
- Biological Abstracts, vol.78,1984,ref.46852
- PLANT PHYSIOLOGY. vol. 93, 1990, ROCKVILLE, MD, USA. pages 1504 - 1520; CATTIVELLI, L., ET AL.: 'Molecular cloning and characetization of cold-regulated genes in barley'
- PLANT PHYSIOLOGY. vol. 89, 1989, ROCKVILLE, MD, USA. pages 577 - 585; PERRAS, M., ET AL.: 'Synthesis of freezing tolerance proteins in leaves, crown, and roots during cold acclimation of wheat'
- JOURNAL OF BIOLOGICAL CHEMISTRY. vol. 266, no. 3, 25 January 1991, BALTIMORE US pages 1564 - 1573; LEAH, R., ET AL.: 'Biochemical and molecular characterization if three barly seed proteins with antifungal properties'
- CHEMICAL ABSTRACTS, vol. 107, 1987, Columbus, Ohio, US; abstract no. 131212, MOHAPATRA, S S., ET AL.: 'Changes in protein patterns and translatable mRNA populations during cold acclimation of alfalfa'
- THE PLANT CELL. vol. 1, April 1989, ROCKVILLE, MD, USA. pages 447 - 457; MAUCH, F., ET AL.: 'Functional implications of the subcellular localization of ethylene-induced chitinase and beta-1,3-glucanase in bean leaves'

## Description

### FIELD OF THE INVENTION

The present invention relates to the identification and isolation of extracellular polypeptides which are associated with plant frost or cold tolerance.

### BACKGROUND OF THE INVENTION

In order to facilitate discussion of already known aspects of frost tolerance in plants and the contribution of the subject invention, several journal articles are referred to herein, in accordance with the following index numbers for the group I and group II listing of references.

### GROUP I

- 1.0: Andersson JA, Ashworth EN (1986) The effects of streptomycin, desiccation and UV radiation on ice nucleation by Pseudomonas viridiflava. Plant
- 1.1: Physiol 80: 956-960. Cutler et al. (1989) J. Plant Physiology 135:351 - 354)
- 1.2: Cattivelli L and Bartels D (1990) Plant Physiol. 93:1504-1510
- 2.: Duman JG, Morris JP, Castellino FJ (1984) Purification and composition of an ice nucleating protein from queens of the hornet, Vespula maculata. J Comp Biochem Physiol B 154: 79-83.
- 3.0: Fischer R, Behnke 8, Apel K (1989) The effect of chemical stress on the polypeptide composition of the intercellular fluid of barley leaves. Planta 178: 61-68.
- 3.1: George et al. (1990) Gene 91:159-165
- 4.: Guy CL (1990) Cold acclimation and freezing stress tolerance: role of protein metabolism. Annu Rev Plant Physiol Plant Mol Biol 41: 187-223.
- 5.0: Guy CL, Haskell D (1987) Induction of freezing tolerance in spinach is associated with the synthesis of cold acclimation-induced proteins. Plant Physiol 84: 872-878.
- 5.1: Guy CL, Neimi KJ and Brambl R (1985) Altered gene expression during cold acclimation of spinach. Proc. Natl. Acad. Sci. USA 82:3673-3677.
- 5.2: Guy CL and Haskell D (1989) Preliminary characterization of high molecular mass proteins associated with cold acclimation in spinach. Plant Physiol. Biochem. 27:777-784.
- 6.: Huner NPA, Macdowall FDH (1976) Chloroplastic proteins of wheat and rye grown at cold hardening temperatures. Can J Biochem 54: 848-853.
- 7.: Kaku S (1973) High ice nucleating ability in plant leaves. Plant Cell Physiol 14: 1035-1038.
- 8.: Kieft TL (1988) Ice nucleation activity in lichens. Appl Environ Microbiol 54: 1678-1681.
- 9.0: Kieft TL, Ruscetti T (1990) Characterization of biological ice nuclei from a lichen. J Bacteriol 172: 3519-3523.
- 9.1: Kurkela and Franck (1990) Plant Molecular Biology 15:137-144.
- 10.: Laemmli UK (1970) Cleavage of structural proteins during the assembly of the head of bacteriophage T4. Nature 227: 680-685.
- 11.: Lindow SE (1983) The role of bacterial ice nucleation in frost injury to plants. Annu Rev Phytopathol 21: 363-384.
- 12.: Lindow SE, Arny DC, Upper CD, Barchet WR (1978a) The role of bacterial ice nuclei in frost injury to sensitive plants. In: Li PH, Sakai A (eds) Plant cold hardiness and freezing stress, vol I. Academic Press, London, New York, pp 249-263.
- 13.: Lindow SE, Arny DC, Upper CD (1978b) Erwinia herbicola: A bacterial ice nucleus active in increasing frost injury. Phytopathology 68: 523-527.
- 14.: Lindow SE, Arny DC, Upper CD (1982) Bacterial ice nucleation: A factor in frost injury to plants. Plant Physiol 70: 1084-1089.
- 15.: Maki LR, Willoughby KJ (1978) Bacteria as biogenic sources of freezing nuclei. J Appl Meteorol 17: 1049-1053.
- 16.: Mauch F, Staehelin LA (1989) functional implications of the subcellular localization of ethylene-induced chitinase and β-1,3-glucanase in bean leaves. Plant Cell 1: 447-457.
- 17.: Meza-Basso L, Albardi M, Raynal M, Ferro-Cardinanos M-L, Delseny M (1986) changes in protein synthesis in rapeseed Brasica napus seedlings during a low temperature treatment. Plant Physiol 82: 733-738.
- 18.: Mohapatra SS, Pcole RJ, Dhindsa RS (1987) Changes in protein patterns and translatable messenger RNA populations during cold acclimation of alfalfa. Plant Physiol 84: 1172-1176.
- 19.: Mohapatra SS, Poole RJ, Dhindsa RS (1988) Detection of two membrane polypeptides induced by abscisic acid and cold acclimation: possible role in freezing tolerance. Plant Cell Physiol 29: 727-730.
- 20.: O'Farrell PH (1975) High resolution two dimensional electrophoresis of proteins. J Biol Chem 250: 4007-4021.
- 21.: Perras M, Sarhan F (1989) Synthesis of freezing tolerance proteins in leaves, crown and roots during cold acclimation of wheat. Plant Physiol 89: 577-585.
- 21.1: Pihakaski S and Antikainen M (1991) Biological Abstracts, Vol. 9 ref. 57717
- 22.: Rajashekar CB, Li PH, Carter JV (1983) Frost injury and heterogeneous ice nucleation in leaves of tuberbearing Solanum species. Plant Physiol 71: 749-755.
- 23.: Robertson AJ, Gusta LV, Reaney MJT, Ishikawa M (1987) Protein synthesis in bromegrass (Bromus inermis Leyss) cultured cells during the induction of frost tolerance by abscisic acid or low temperature. Plant Physiol 84: 1331-1336.
- 24.: Southworth MW, Wolber PK, Warren GJ (1988) Nonlinear relationship between concentration and activity of a bacterial ice nucleation protein. J Biol Chem 263: 15211-15216.
- 25.: Warren GJ (1987) Bacterial ice nucleation: molecular biology and applications. Biotechnol Genet Eng Rev 5: 109-135.
- 26.: Wolber P, Warren G (1989) Bacterial ice nucleation proteins. Trends Biochem Sci 14: 179-182.

### GROUP II

- 1.0: Abeles, F.B and Forrence L.E. (1970). Plant Physiology 45:395-400.
- 1.1: Ashworth, E.N. Plant Physiol. 92, 718-725 (1990).
- 2.: Blum, A. CRC Crit. Rev. Plant Sci. 2, 199-238 (1985).
- 3.: Chakrabartty, A., Yang, D.S.C. & Hew, C.L. J. Biol. Chem. 264, 11313-11316 (1989).
- 4.: Davies, P.L. & Hew, C.L. FASEB J. 4, 2460-2468 (1990).
- 5: DeVries, A.L. Meth. Enzymol. 127, 293 (1986).
- 6.: Duman, J.G., Xu, L., Neven, L.G., Tursman, D. & Wu, D.W. in Insects at Low Temperature, R.J. Lee, Jr. and D.L. Denlinger, Eds. (Chapman and Hall, New York, 1991), pp. 94-127.
- 7.: Feeney, R.E. Comments Agric. & Food Chemistry 1, 147-181 (1988).
- 8.0: Fourney, R.M., Joshi, S.B. & Hew, C.L. Can. J. Zool. 62, 28-33 (1983).
- 8.1: Harlow, E. & Lane D. Antibodies, A Laboratory Manual. Cold Spring Harbor Laboratory, Cold Spring Harbor New York (1988).
- 9.: Hew, C.L., Slaughter, D., Joshi, S.B., Fletcher, G.L. & Ananthanarayanan, V.S.J. Comp. Physiol. B 155, 81-88 (1984).
- 10.: Knight, C.A. & Duman, J.G. Cryobiology 23, 256-262 (1986).
- 11.: Krol, M., Griffith, M. & Huner, N.P.A. Can.J.Bot. 62, 1062-1068 (1984).
- 12.: Mauch, F. & Staehelin, L.A. The Plant Cell 1, 447-457 (1989).
- 13.: Parody-Morreale, A., Murphy, K.P., Di Cera, E., Fall, R., DeVries, A.L. & Gill, S.J. Nature 333, 782-783.
- 14.: Pearce, R.S. Planta 175, 313-324 (1988).
- 15.: Sakai, A. & Larcher, W. Frost Survival of Plants (Springer-Verlag, Berlin, Heidelberg, 1987), pp. 1-38.
- 16.: Storey, K.B. & Storey, J.M. Physiol. Rev. 68, 27-84 (1988).
- 17.: Tomchaney, A.P., Morris, J.P., Kang, S.H. & Duman, J.G. Biochemistry 21, 716-721 (1982).
- 18.: Uemura, M. & Steponkus, P. Plant Physiol. 91, 1131-1137 (1989).
- 19.: Wu, D.W., & Duman, J.G. J. Comp. Physiol. B 161, 279-283 (1991).
- 20.: Wu, D.W., Duman, J.G. & Xu, Lei. Biochim. Biophys. Acta 1076, 416-420.

Low temperature is a major environmental limitation to the production of agricultural crops. For example, late spring frosts delay seed germination, early fall frosts decrease the quality and yield of harvests and winter low temperatures decrease the survival of overwintering crops, such as winter cereals and fruit trees. However, some plants have the ability to withstand prolonged subfreezing temperatures. If proteins involved in the development of frost tolerance in these plants, as well as the corresponding genes, can be identified, it may be possible to transform frost sensitive crop plants into frost tolerant crop plants and extend the range of crop production.

Biological organisms can survive icy environments by inhibiting internal ice formation. This strategy requires the synthesis of antifreeze proteins (AFPs) or thermal hysteresis proteins (THPs). Four distinct types of (AFPs) have been identified in fish (P.L. Davies & C.L. Hew. (II-4) and a number of different THPs have been identified in insects. These previous findings suggest that this adaptive mechanism has arisen independently in different organisms. Antifreeze proteins are thought to bind to ice crystals to prevent further growth of the crystals. The presence of antifreeze proteins can be determined (1) by examining the shape of ice crystals as they form and (2) by measuring the existence of thermal hysteresis (the difference in temperature at which a particular solution melts and freezes).

It was generally understood that antifreeze proteins did not exist in plants. Altough work was conducted to determine peptide characteristics which might be expressed in plant tissue as extracted from cold acclimated plants (I-1.2, 1-21, I-21.1). It was thought that some internal mechanism of the plant cells adapted them to withstand external ice crystal formation on their outer call without damaging the cell. Kurkela and Franck (I-9.1) recently reported that a plant gene expressed at low temperature codes for a protein similar in amino acid sequence to the antifreeze protein of Davis et al (II-4). Kurkela et al, did not have sufficient amounts of the encoded protein to determine whether it exhibited an antifreeze activity in the plant and particularly within the plant cell. Cutler et al. (I-1.1) used fish antifreeze protein to demonstrate that the presence of antifreeze protein can increase frost tolerance in plants. George et al. (I-3.1) transformed corn protoplasts with a synthetic gene for the flounder antifreeze protein in an attempt to use antifreeze proteins for improving plant cold hardiness.

Guy et al. (I-5.1) discusses a rapid and stable change in the translatable poly(A)⁺ RNA populations extracted from leaves of plants exposed to low temperatures. Total protein analysis of the plant tissues was conducted to detect proteins which might be associated with frost tolerance in plants. Proteins found in cold acclimated leaf extracts having molecular weights of 110 kd, 82 kD, 66 kD, 55 kD and 13 kD were not found in non-acclimated leaf extracts. It is thought that the increased expression of certain mRNAs may encode proteins that are involved directly in a development of increased freezing tolerance for the plant. Guy et al. (I-5.2) characterizes high molecular mass proteins which are believed to be associated with cold acclimation in spinach. As with Guy et al. (I-5.1) the total protein content of the acclimated spinach leaf is assessed. Cold acclimated proteins having molecular weights of 110 kD, 90 kD and 79 kD were identified. However, their location and function within the cell remain unknown.

### SUMMARY OF THE INVENTION

It was the general impression that the mechanism responsible for frost tolerance resided within the cell so as to protect it internally from ice crystals which formed usually on the outside of the cell. No one had given any thought to the possibility of the existence of antifreeze proteins in plants and that, in addition to antifreeze proteins, ice nucleation proteins may also be present in the plant. Furthermore, no thought had been given to the possibility that such proteins could be located outside of the cell to effect an entirely different mechanism for protecting the plant from freezing. Quite surprisingly, we have found that a plurality of polypeptides occur extracellularly and provide for ice nucleation, antifreeze properties by controlling of ice crystal growth in the extracellular spaces and enzymatic activity, which adapts the plant cell wall to conform to the protoplast during formation of ice crystals, to retain plant cell viability upon plant thawing. These extracellular polypeptides are located in the extractable portion of the plant apoplast, which includes the outer surface of the plasmalemma, the region between the plasmalemma and the cell wall, the cell wall, the middle lamella, the intercellular spaces and the tracheids and vessels of the xylem. It is understood that throughout this specification, the term extracellular polypeptides has the above meaning.

According to an aspect of the invention, antifreeze polypeptides common to frost tolerant plants are provided. The polypeptides are located in the extracellular space to control ice crystal growth in the xylem and intercellular plant space, the control of ice crystal growth providing a degree of plant frost tolerance. The polypeptides associated with such antifreeze properties are selected from a group of polypeptides having respectively apparent molecular weights as based on SDS-PAGE gel analysis of about 5 kD, about 9 kD, about 11 kD, about 22 kD, about 24 kD, about 30 kD and about 35 kD.

According to another aspect of the invention, a plurality of polypeptides derived from intercellular spaces of plant cells having frost tolerance is provided. Some of the polypeptides are ice nucleators for developing ice crystals in extracellular spaces of plant tissue, some of the polypeptides are antifreeze components which control ice crystal growth in extracellular spaces and some of the polypeptides are enzymes which adapt plant cell walls to function differently during formation of ice crystals in plant intercellular spaces.

According to a preferred aspect of the invention, the group of polypeptides has molecular weights of about 5 kD, about 9 kD, about 11 kD, about 22 kD, about 24 kD, about 30 kD, about 36 kD, about 60 kD and about 68 kD.

According to another aspect of the invention, a process for extracting polypeptides exhibiting ice nucleating properties, ice crystal growth controlling properties and enzymatic plant cell wall modification properties, comprises:
i) infiltrating cut leaves of plants having frost tolerance, with a polypeptide extraction medium,
ii) removing the extraction medium from the leaves without rupturing plant cells, and
iii) recovering the polypeptides from the extraction medium.

According to another aspect of the invention, antibodies to one or more of the aforementioned polypeptides may be developed, such antibodies being optionally adapted for detection in an immunoassay for determining if a plant is frost tolerant.

According to another aspect of the invention, frozen food preparations may include one or more of the above polypeptides and in particular ice cream and fruit preparations which include one or more of the polypeptides to provide a superior product having minute crystalline structure. In addition, the polypeptides are useful in the cryopreservation of biological tissues.

According to various aspects of the invention, the polypeptides have been characterized by their apparent molecular weights based on their migration in SDS-PAGE gels relative to known molecular weight markers. It is appreciated that the polypeptides of this invention may migrate differently in different types of gels, particularly for different concentrations of acrylamide in the gel. Hence, the molecular weight characterization of the polypeptides of this invention is intended to cover the equivalent polypeptides as they might have slightly different molecular weights on different gels.

### BRIEF DESCRIPTION OF THE DRAWINGS

Preferred embodiments of the invention shall be discussed with respect to the drawings wherein.

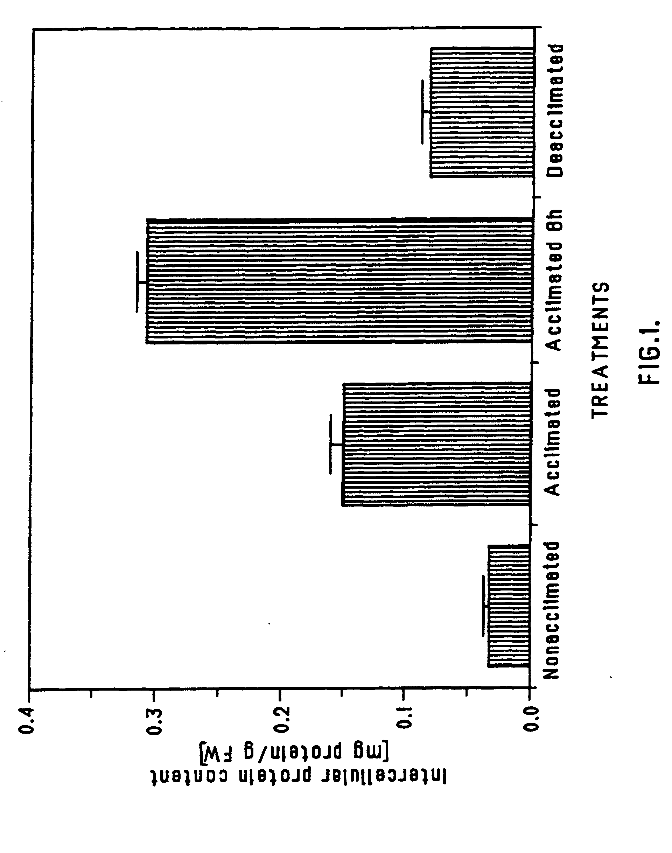

Figure 1 depicts concentrations of proteins from the extracellular spaces of winter rye leaves grown under various temperature regimes;

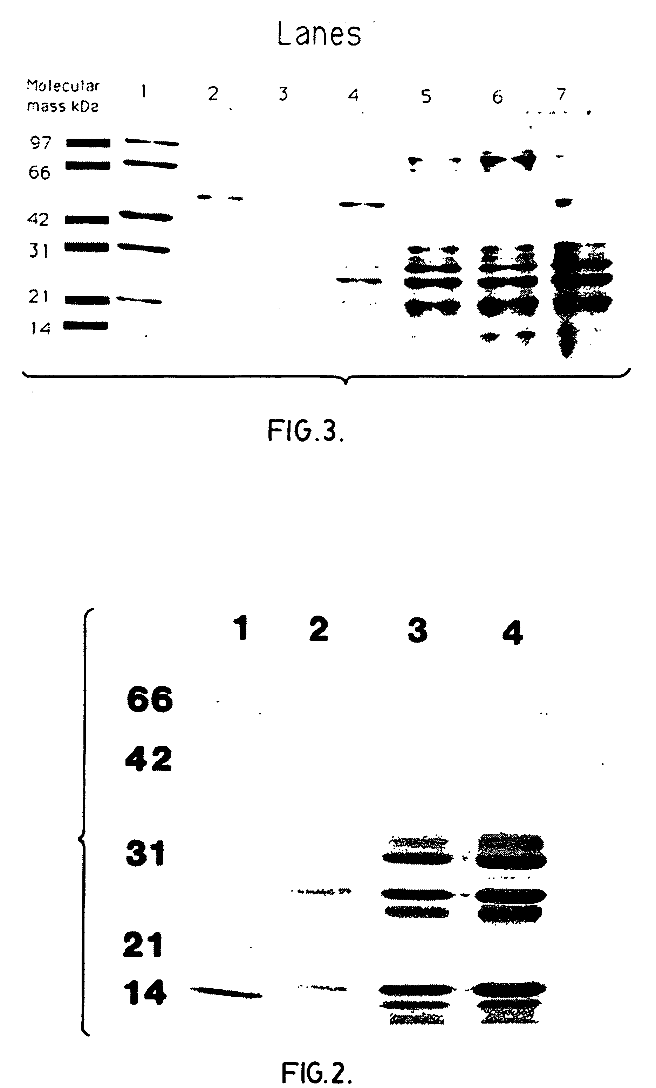

Figure 2 depicts an SDS-PAGE of extracellular space proteins isolated from winter rye leaves grown under various temperature regimes where lane 1, molecular mass markers; lane 2, extracellular polypeptides from rye plants grown at 20/16°C (day/night) with a 16 hour day; lane 3, extracellular polypeptides from rye plants grown at 5/2°C with a 16 hour day; lane 4, extracellular polypeptides from rye plants grown at 5/2°C with an 8 hour day;
Figure 3 depicts an SDS-PAGE of extracellular space polypeptides isolated from cold acclimated winter rye leaves grown with an 8-hour daylength at different stages of development where lane 1, molecular mass markers; lane 2, extracellular polypeptides from rye plants grown at 20/16°C with a 16 hour day for 7 days; lane 3, extracellular polypeptides from rye plants (20/16°C, 7 days old) transferred to 5/2°C with an 8 hour day for 28 days; lane 4, extracellular polypeptides from rye plants transferred to 5/2°C for 43 days; lane 5, extracellular polypeptides from rye plants transferred to 5/2°C for 50 days; lane 6, extracellular polypeptides from rye plants transferred to 5/2°C for 71 days; lane 7, extracellular polypeptides from rye plants transferred to 5/2°C for 95 days;

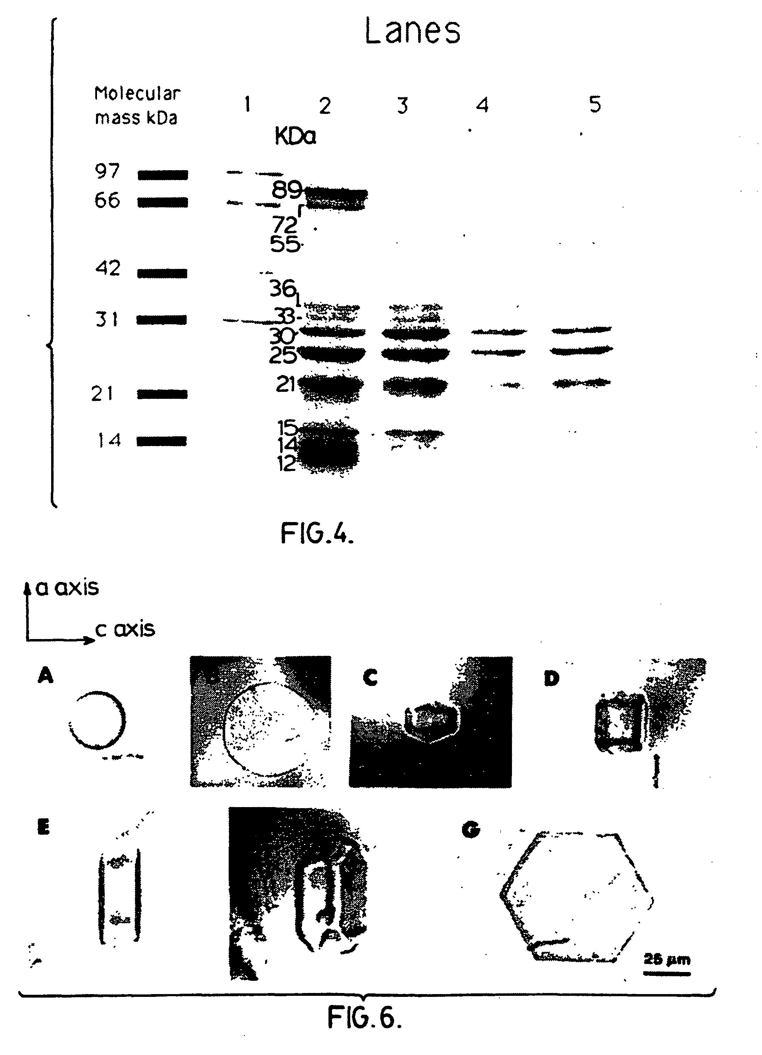

Figure 4 depicts an SDS-PAGE of extracellular space proteins isolated from deacclimating winter rye leaves where lane 1, molecular mass markers; lane 2, extracellular polypeptides from plants grown at 20/16°C for 7 days and then transferred to 5/2°C with an 8 hour day for 42 days; lanes 3, 4 and 5, extracellular polypeptides from plants grown as described in lane 2 and then transferred to 20/16°C with an 16 hour day for 4, 6 and 8 days, respectively;

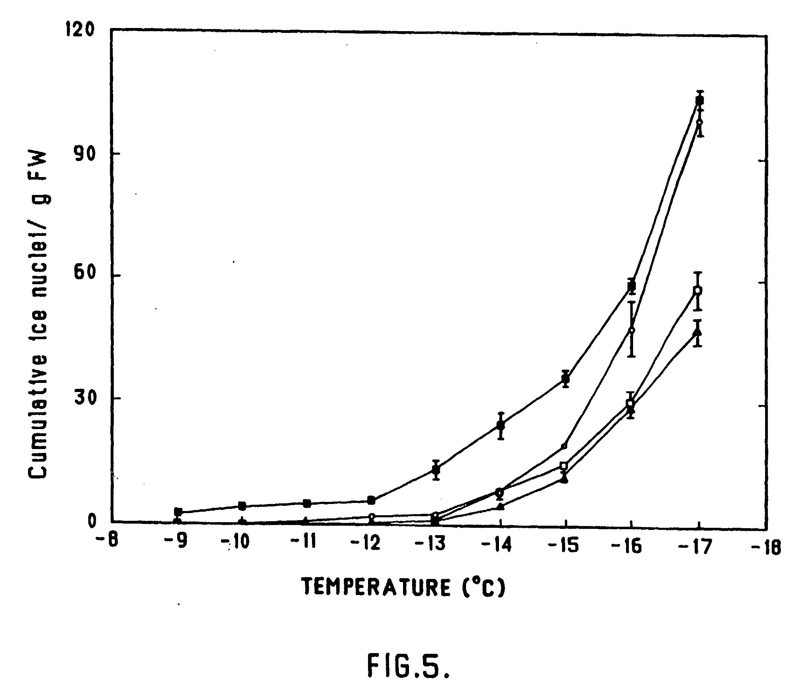

Figure 5 depicts the ice nucleation activity of various ultrafiltered extracellular extracts from rye leaves grown under different temperature regimes;

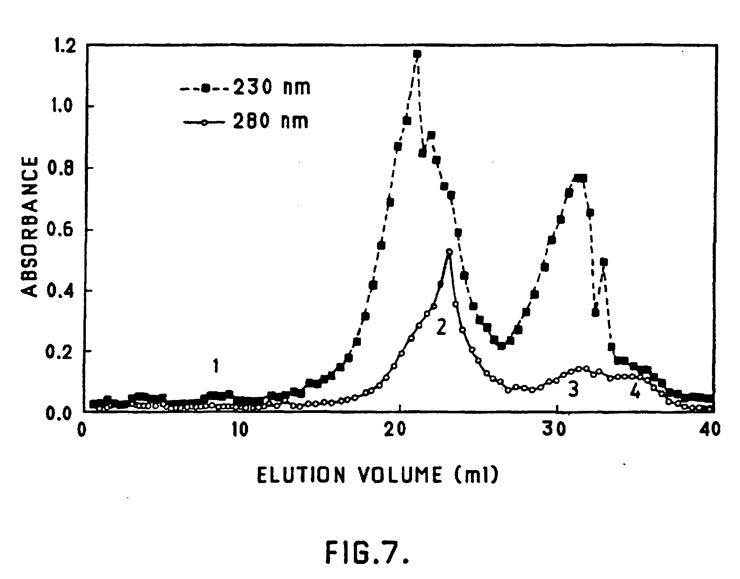

Figure 6 illustrates the antifreeze activity in extracellular extracts of cold acclimated winter rye leaves. Antifreeze activity was determined by observing ice crystal morphology using a nanoliter osmometer (Clifton Technical Physics, Hartford, N.Y., U.S.A.) (II-5). Orientation of the ice crystals in C, D, E, F: the a-axis represents growth in the basal plane and the c-axis represents growth normal to the basal plane;
Figure 7 illustrates fractionation of extracellular extracts from cold acclimated leaves by column chromatography;

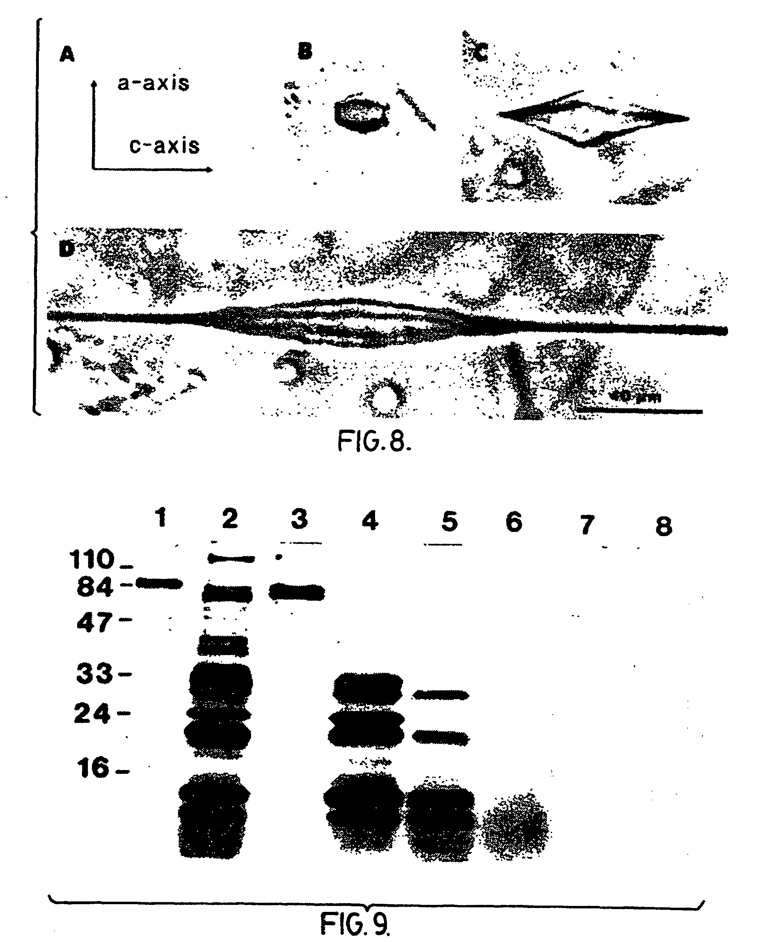

Figure 8 illustrates the ice crystal morphology of partially purified and concentrated antifreeze protein from cold acclimated winter rye leaves. (A) Orientation of the crystal as described in Fig. 6. (B, C & D) Growth sequence of an ice crystal as the temperature was lowered. (B) Incomplete bipyrimid; (C) bipyrimid; (D) needle-like; and
Figure 9 is an SDS-PAGE of polypeptides associated with column fractions of each 280 nm peak shown in Fig. 8.

### DETAILED DESCRIPTION OF THE PREFERRED EMBODIMENTS

The novel polypeptides which we have discovered and which are associated with plant frost tolerance are of 3 categories:
i) ice nucleation polypeptides,
ii) ice crystal growth control polypeptides, and
iii) polypeptides for enzymatic modification of cell wall.

The purpose of the ice nucleation proteins is to initiate ice crystal formation in the plant tissue when the plant tissue is exposed to freezing temperatures. By virtue of the ice nucleation proteins being located in the extracellular plant spaces, crystal growth is initiated in the intercellular spaces between the cell wall. The antifreeze proteins are also located outside the plasma membrane to control and limit crystalline growth in the intercellular spaces so as to not exert pressures on the cell membrane which would cause rupture thereof. The enzymes present in extracellular spaces function to increase the flexibility of the cell wall material to allow the cell wall to conform to the protoplast without damaging cellular material and retaining cell viability upon thawing of the plant tissue. In the process of freezing the water in the plant tissue water is allowed to migrate through the cell walls into the intercellular spaces where the water is allowed to freeze under controlled conditions in forming the intercellular ice crystals.

It is believed that the proteins associated with frost tolerance are made endogenously by the plant cells and are secreted through the plasma membrane into the intercellular spaces to effect and modify ice crystal formation during freezing temperatures. It is understood that the make up of the frost tolerant proteins may comprise one or more of the identified polypeptides. More than one of the identified polypeptides may combine to provide a protein structure which provides one of the noted frost tolerant properties of ice nucleation, antifreeze or enzymatic action.

We have found that the polypeptides of the frost tolerant proteins are produced to a lesser extent by plant cells even at warmer temperatures such as 20°C. With the correct environmental conditions, production of the polypeptides associated with frost tolerance is dramatically increased as the plant is subjected to conditions which resemble early spring or late fall when frost can set in. To our knowledge this is the first finding of frost tolerance-inducing polypeptides being located in extracellular spaces of plant tissue. In view of our having located the subject polypeptides in intercellular spaces we have developed a process for extracting the polypeptides from those spaces. Generally, the process is two-step and includes:
i) severed or cut leaves are vacuum infiltrated with an extraction buffer preferably containing EDTA, magnesium chloride or calcium chloride, ascorbate, mercaptoethanol and protease inhibitors, and
ii) extracting the infiltrate from the plant tissue while the cells remain unbroken. The recovered extract exhibits ice nucleation activity and glucanase activity as well as antifreeze activity. Preferably ice nucleation activity can be measured by the droplet freezing assay. The ice nucleation activity decreases upon addition of sulfhydryl reducing agent such as dithiothreitol and mercaptoethanol in the manner to be discussed with respect to the examples. The antifreeze activity of the extract was determined by observing ice crystal formation on a freezing stage mounted on a light microscope. In the presence of the extract the ice crystals form bipyrimidal and hexagonal structures which indicate control in the crystalline growth. Such structures are similar to those formed in the presence of other types of antifreeze proteins isolated from other sources such as the sea raven fish (II-4). It has been found that the addition of protease to the extract eliminates the antifreeze activity which indicates the presence of a protein. The glucanase activity is measured as the enzymatic release of glucose equivalents from soluble laminaran (poly-beta-1,3-glucose. The glucanase is more active in the presence of calcium.

It is understood that various separation techniques may be employed which remove the infiltrate from the intercellular spaces without rupturing the plant cells. Such techniques include vertically orienting the leaves in a funnel placed inside a centrifuge tube. Such vertical layering avoids severe bending of the leaves. The leaves are then centrifuged to recover the infiltrate without rupturing the cells. Other techniques are available for polypeptide extraction. For example, leaves may also be extracted by perfusion with appropriate extraction solutions. The extracellular polypeptides are water-soluble and are found in the total soluble fraction when plant tissues are homogenized.

The frost tolerance inducing polypeptides are beneficial to any type of plant where intercellular ice formation can be initiated and ice crystal growth controlled. Any plant tissue can, in a variety of ways, be adapted to provide or include these polypeptides so that they can withstand lower freezing temperatures and hence, are more likely to survive harsher climates or provide at least prolonged growing periods in the later fall and earlier growth in the early spring. It has also been found that in some species of plants supercooling of the plant liquids may be achieved at temperatures below - 35°C. It is possible that the antifreeze polypeptides are produced in the absence of any nucleating peptides. Such antifreeze peptides serve to supercool the plant liquids to permit survival of the cell.

As can be appreciated, the frost tolerant proteins as provided by this invention have a variety of uses. A significant use is in the detection of frost tolerant characteristics in plants. Antibodies may be developed to one or more of the polypeptides and by way of an immunoassay other plants can be tested for the presence of polypeptides to determine their frost tolerance capability. It is also understood that plants could be transformed with genetic information which encodes for the subject polypeptides to improve or provide frost tolerance in other types of plants. Furthermore, the polypeptides would be useful in the cyropreservation of biological tissues. Polypeptides also have a broad application in improving the quality of frozen foods and in particular, ice cream and frozen fruit. The use of the polypeptides would induce minute crystalline structure in the ice and prevent recrystallization to produce a superior product.

Further aspects of the invention will be understood based on the following specific discussion and examplification of the invention.

### EXPERIMENTAL PROTOCOL

### Production of Plant Material

Winter rye seeds (Secale cereale L. cv. Musketeer) were sown in 15 cm plastic pots containing coarse vermiculite and germinated for one week at 20:16°C (day:night) with a 16 hour daylength. Plants transferred to a at 5:2°C (day:night) and a light regime of 16:8 h (day:night) are referred to as cold acclimated rye (RH). Plants grown at 5:2°C (day:night), but with a light regime of 8:16 h (day:night) are referred to as cold acclimated rye - short day (RH-SD). The pots that remained in the growth chamber at 20°C for another three weeks are control or nonacclimated rye plants (RNH). Rye plants that were grown at 5:2°C (day:night) (8 h day:16 H night) for exactly seven weeks and then were shifted to the growth chamber at 20°C for four days are referred as deacclimated (Deacc). All plants were watered with modified Hoagland nutrient solution as described by Huner and Macdowall (I-6).

### Protein Extraction

Extracellular proteins were removed from the leaves of RNH, RH, RH-SD and Deacc plants. In each instance the extracellular extracts were prepared by vacuum infiltration of the leaves with 5 mm EDTA, 10 mm ascorbic acid, 10 mm mercaptoethanol, 1 mm phenylmethyl sulfonylfluoride, 2 mm caproic acid and 2 mm benzamidine. The vacuum infiltration is in accordance with the process described in Mauch and Staehelin (I-16). The treated leaves were packed vertically in a funnel placed in a centrifuge tube so as to avoid bending of the leaves. With the leaves packed in the funnel the material was centrifuged to remove without rupturing the cells of the leaves the extracellular infiltrate which is captured in the centrifuge tube as an extract.

### Protein Electrophoresis

For the results presented in Figures 2, 3 and 4, the extraceullar proteins were precipitated from extracellular extracts for purposes of electrophoresis by the addition of 1.5 volumes of 1% acidic acid in methanol and incubating overnight at -20°C. The protein pellet was washed with 100% ethanol and 70% ethanol at 5°C and then dried in a desiccator. The protein was resuspended in Laemmli (I-10) sample buffer [60 mM Tris-HCl, pH 6.8; 10% glycerol; 2% sodium dodecyl sulfate (SDS); 5% mercaptoethanol] and separated by electrophoresis, along with Biorad unstained standards, on 10% acrylamide gels using 90 V for the stacking gel and 110 V for the separating gel (I-10). The gels stained were Coomassie blue. For the results presented in Figure 9, proteins present in column fractions of the extracellular extracts were solubilized directly in Laemmli sample buffer (I-10), separated by electrophoresis, along with Biorad prestained standards, on 13.5% acrylamide gels at 200 V. The gels stained with ammoniacal silver.

### Quantification of Extracellular Proteins

Extracellular proteins were extracted from RNH, RH, RH-SD and Deacc rye leaves, as outlined above in "Protein Extraction". Protein concentration of the different extracts was determined by the Bio-Rad method with BSA as the standard. At least four replicates were run to obtain an accurate estimate of the extractable extracellular protein content in the different types of leaves. The extracellular extracts from leaves were subjected to ultrafiltration through an Amicon minicon membrane to concentrate the extracts approximately ten times and to remove compounds that interfered with INA assay (I-13). The protein concentration of all extracts after ultrafiltration was again determined by the Bio-Rad method. Concentrated extracts were used in the droplet freezing technique to determine the spectrum of active ice nuclei within a given temperature range.

### Polypeptides extracted from the Extracellular space of rye leaves:

Different amounts of proteins can be extracted from the extracellular space by the various treatments. Nonacclimated leaves (hardy to -12°C) had an extracellular protein content averaging 0.034 mg protein/g fresh weight. This amount of protein increased when leaves were allowed to develop at 5°C with either a daylength of 16 hours (hardy to -19°C), protein content of 0.149 mg/g fresh weight, or a daylength of 8 hours (hardy to -30°C), protein content of 0.307 mg/g fresh weight. Thus there is a 9 fold increase in extracellular proteins in rye plants grown at 5°C with a short daylength as compared with the protein levels in nonacclimated rye plants grown at 20°C. These protein levels decreased when the leaves were shifted back to 20°C to acclimate (Figure 1).

The protein profile of extracellular extracts shows remarkable changes between the different types of leaves. Using 10% acrylamide gels, the SDS-PAGE electrophoresis analysis of the intercellular fluid revealed the presence of at least 12 polypeptides. Two of these extracellular polypeptides with molecular masses of 77 and 73 kDa were observed in extracellular fluids from cold acclimated leaves only (Figure 2; lanes 3 and 4). The 77 and 73 kDa polypeptides stained red with Coomassie blue. Increases of eight polypeptides with molecular masses of 36, 33, 30, 25, 21, 15, 14 and 13 kDa were observed in extracellular fluids from acclimated leaves (Figure 2). Increases in two polypeptides with molecular masses of 23 and 20 kD were observed only in leaves cold-acclimated with a short day.

To further characterize the polypeptides of the intercellular spaces, a time course study was carried out with the aim of correlating the appearance of these polypeptides with the development of the freezing tolerance. Most of the polypeptides of the intercellular spaces were detected at very low levels in nonacclimated leaves (Figure 3, lane 2). The polypeptides accumulated steadily during cold acclimation for 35, 50, 57 and 78 days (Figures 3, lanes 3, 4, 5 and 6). At 78 days, rye leaves cold acclimated with a short day are maximally frost tolerant, exhibit the highest levels of all extracellular polypeptides and exhibit a new polypeptide at 109 kD. After cold acclimation for 102 days, the 109, 77 and 73 kDa polypeptides were no longer present and the leaves are less frost tolerant (Figure 3, lane 7). This finding suggests that the appearance of most of these 13 polypeptides in the extracellular space are correlated with changes in frost tolerance. The extracellular protein profile was also monitored during deacclimation by transferring cold acclimated plants at their hardiest stage to a 20°C environment for different lengths of time. As shown in Figure 4, the levels of most of the 12 intercellular polypeptides (lane 2, maximally cold-acclimated) were greatly reduced following 4 days of deacclimation (lane 3) and continued to decline steadily after deacclimation for 6 and 8 days (lanes 4,5). The molecular weight markers are in the left hand column which are understood to approximate the molecular weights of the polypeptides in lane 1. The column of molecular weight between lane 1 and lane 2 is believed to be more accurate.

To verify that the presence of these extracellular space polypeptides are essential to the induction of freezing tolerance and not merely a result of low-temperature exposure, freezing studies were carried out on the extracellular extracts from different types of leaves (Figure 5). Extracellular extracts from cold acclimated rye leaves grown under a short photoperiod initiate ice formation at -9°C (■), whereas extracts from cold-acclimated rye leaves grown under a long photoperiod initiate ice formation at -10°C (○). The extracts from nonacclimated (□) and deacclimated leaves (Δ) initiate ice formation at the lowest temperature (-13°C). The difference in ice nucleation activity of the extracellular extracts between nonacclimated and cold acclimated leaves (Figure 5) may be attributed to the fact that acclimated leaves maintain higher levels of proteins in the extracellular spaces (Figure 1). The effect of protein concentration was examined by using ultrafiltration to obtain nonacclimated and cold acclimated extracellular extracts that were equal in protein content. When ice nucleation activity was assayed and calculated for the two extracts, a striking increase in the cumulative number of ice nuclei per gram fresh weight was found in cold acclimated leaves. The number of ice nuclei per gram fresh weight (mean ± standard deviation) at -15°C was 2268.7 ± 292.1 in intercellular extracts from nonacclimated leaves and 7047.6 ± 916.6 in extracts from cold acclimated rye leaves grown under a long daylength. A statistically significant increase in the number of ice nuclei per gram fresh weight was found in the extracts of cold acclimated rye leaves between the two extracts as determined by a Student's t test (a = 0.01, n = 4). The low threshold temperature for nucleation suggests that the ice nucleators present in the extracts are not intact ice nucleation sites.

### Antifreeze Activity of Extraceullar Proteins

Extracellular proteins as extracted in accordance with the above technique were evaluated with respect to antifreeze activity. The antifreeze activity was determined by observing ice crystal morphology using a nanoliter osmometer (Clifton Technical Physics, Hartford, N.Y., U.S.A.) (II-3). Orientation of the ice crystals in Figures 7 C, D, E, F: the a-axis represents growth in the basal plane and the c-axis represents growth normal to the basal plane. (A) is an ice crystal formed in distilled water, oriented so that the c-axis is perpendicular to the plane of the paper. (B) is an ice crystal formed in presence of extracellular extract of non-acclimated plant. (C, D, E & F) are growth sequences of an ice crystal in the presence of crude extracellular extract from cold acclimated winter rye leaves as the temperature was lowered. (G) is a hexagonal column of ice as shown in (F) reoriented so that the c-axis is perpendicular to the plane of the paper.

### Fractionation of proteins of extracellular extracts

Extracellular extracts were concentrated five-fold, exchanged into 50 mM NH₄HCO₃ by ultrafiltration (Centriprep-10, Amicon Canada Ltd., Oakville, ON, Canada) and applied to a Sephacryl 200 (Pharmacia LKB Biotechnology, Uppsala, Sweden) column (0.5 x 32 cm) in 50 mM NH₄HCO₃. The eluate was monitored for UV absorbance at 280 nm (O -- O) and 230 nm **(**_{**-**} **--** _{**-**}**)**. Proteins standards were eluted separately to estimate protein size. Ferritin, 440 kD, eluted at 9.5 ml; aldolase, 158 kD, eluted at 11.5 ml: bovine serum albumin, 67 kD, eluted at 13.5 ml: and trypsinogen, 24 kD, eluted at 16.5 ml. As shown in Figure 7, four peaks were observed with apparent molecular masses of 305 kD (peak 1), 5 kD (peak 2), 2 kD (peak 3) and < 1 kD (peak 4). Only fractions associated with peak 2 formed hexagonal and bipyrimidal ice crystals upon testing fraction antifreeze properties.

### SDS-PAGE of polypeptides

The polypeptides associated with column fractions of each of the peaks for 280 nM as shown in Figure 7 were evaluated. The 13.5% acrylamide gel was silver-stained as shown in Figure 9. Lane 1 is prestained molecular mass standards; lane 2 is crude intercellular extract; lane 3 is polypeptides eluted at 8 ml (peak 1); lane 4 is polypeptides eluted at 18 ml (shoulder of peak 2); lane 5 is polypeptides eluted at 22 ml (peak 2); lane 6 is polypeptides eluted at 26 ml (shoulder of peak 2); lane 7 is polypeptides eluted at 31 ml (peak 3); and lane 8 is polypeptides eluted at 35 ml (peak 4).

### Further characterization of polypeptides

Winter rye (Secale cereale cv. Musketeer) is an overwintering, herbaceous monocot that can survive temperatures as low as -35°C in the field. Rye leaves survive low freezing temperatures by restricting ice to intercellular spaces (II-2). In this experiment, rye seeds were allowed to germinate at 20°C for one week and the plants then were transferred, either to 20°C for 3 weeks (nonacclimated) or to 5°C for 7 weeks, to induce cold acclimation. Under these growth conditions, leaves from nonacclimated plants withstand freezing to -12°C, whereas cold-acclimated leaves can tolerate -22°C (II-11). Secreted proteins were extracted from the extracellular spaces of winter rye leaves by vacuum infiltration with intercellular washing fluid, followed by centrifugation to recover the infiltrate (II-12). This crude infiltrate was assayed for antifreeze activity by observing the morphology of ice crystals formed in solution using a nanoliter osmometer (II-3,5). In pure water, ice normally grows parallel to the basal plane (a-axes) of the crystal lattice with little growth perpendicular to the basal plane (the c-axis), so that the ice crystals appear flat and round (II-5) (Fig. 6A). In contrast, low (nM) concentrations of antifreeze proteins preferentially inhibit the a-axis growth of ice so that the hexagonal prism faces of the crystal are expressed (II-6) (Fig. 6G). At higher concentrations (*µ*M) of antifreeze protein, the crystals grow predominantly along the c-axis to form hexagonal bipyrimids (II-6) (Fig. 8C).

In this experiment, extracellular extracts of nonacclimated rye leaves froze like distilled water; i.e., only thin, round ice crystals were observed (Fig. 6B). In contrast, all crude extracts of the extracellular space of cold-acclimated winter rye leaves formed hexagonal ice crystals upon freezing (Figs. 6C to 6G). As the temperature was lowered, the crystals expanded first along the c-axis to form incomplete hexagonal bipyrimids (Fig. 6C) and then along the a-axis to form both hexagonal columns (Fig. 6D) and larger hexagonal plates of ice (Figs. 6E to 6G). The formation of hexagonal ice and growth of the ice crystals along the c-axis indicate that antifreeze activity is present in these crude extracts of winter rye (II-3, 5).
Furthermore, the fact that these effects on ice crystal morphology were lost when extracellular extracts from cold-acclimated rye leaves were incubated with 5% (w/v) Streptomyces griseus protease (Sigma Chemical Co., St. Louis, MO, U.S.A.) at 22°C for one hour suggests that the antifreeze activity in winter rye is derived from a protein.

### Antifreeze Mechanism

Antifreeze proteins lower the freezing temperature of a solution noncolligatively by binding to ice crystals and inhibiting crystal growth, but the proteins alter the melting temperature of the solution only by colligative effects (II-5). This thermal hysteresis (the difference between freezing and melting temperatures) is determined by observing the effect of temperature on the growth of a single ice crystal. Melting occurs when faces of the ice crystal become round; freezing occurs when the ice crystal elongates along its c-axis (II-5).

In order to demonstrate thermal hysteresis, we used ultrafiltration, followed by size fractionation on a Sephacryl 200 column, to partially purify proteins contained in the extracellular extracts from cold-acclimated leaves. We obtained four peaks of absorbance at 280 nm with apparent molecular masses of 305, 5, 2 and <1 kD (Fig. 7). These molecular sizes are inaccurate, possibly because the proteins interact with the Sephacryl and so their elution is retarded and they appear smaller in size than they are. Only fractions containing the second (5 kD) peak (Fig. 7) formed bipyrimidal ice crystals in the antifreeze assay. Column fractions exhibiting both absorbance at 280 nm and antifreeze activity (peak 2) were pooled, lyophilized and resolubilized in distilled water for the determination of thermal hysteresis. At this higher protein concentration, ice crystal growth was inhibited along the a-axis (Figs. 8B to 8D). Furthermore, the ice crystals spiked along the c-axis (Fig. 8D) at an average freezing temperature of -1.10°C for five ice crystals. The average melting temperature was -0.78°C, and so the thermal hysteresis was calculated to be 0.33 ± 0.06°C (mean ± S.D., n = 5). Thus, winter rye leaves produce antifreeze protein that has the ability to modify the normal growth pattern of ice and to depress the freezing temperature of a solution noncolligatively.

The thermal hysteresis exhibited by the winter rye antifreeze protein is smaller than that observed for other antifreeze proteins such as found in polar fish (approximately 0.6°) (II-7) or in insects (5°C) (II-7, II-8). This may be due to the fact that the antifreeze proteins from winter rye are not completely purified or to a difference in structure and function.

### SDS-PAGE Analysis of Antifreeze Polypeptides

The results shown in Figure 9 by SDS-PAGE (13.5% acrylamide), demonstrate that the peak 2 fractions of Figure 7 with antifreeze activity contain seven major polypeptides ranging in size from 5 to 36 kD (Fig. 9, lane 4 and Table 1). In addition to the polypeptides exhibiting antifreeze activity, the 30 kD polypeptide is also an endoglucanase. The 30 kD band sometimes appears as a 32 kD band, known to be an endoglucanase precursor. The seven major winter rye polypeptides found in column fractions exhibiting antifreeze activity (Fig. 10, lane 4) are relatively enriched in glycine, asparagine or aspartate, alanine, glutamine or glutamate and serine (see Table 1 for six of the seven polypeptides) but do not contain hexosamines (within the limits of detection by amino acid analysis after 4 h hydrolysis of 15 picomoles of each polypeptide). None of the polypeptides exhibits the high alanine content characteristic of antifreeze glycoproteins and type I antifreeze proteins (II-10). Instead, the rye polypeptides exhibit high hydrophilic amino acid contents, as observed in sea raven and ocean pout (II-4), and also contain the high glycine content observed in some insect antifreeze proteins (II-17) (Table 1).

When proteins are eluted off the Sephacryl column, low ice nucleation activity is detected in peak 1 and peak 4, of Figure 7, with higher levels of activity observed in peak 3. SDS-PAGE separated out two polypeptides at 60 and 68 kD. The ice nucleating protein can be one or a combination of these two polypeptides. These two polypeptides are distinct from the 77 and 73 kD polypeptides shown in Figures 2, 3 and 4 because the 60 and 68 kD polypeptides stain blue with Coomassie blue.

### N-Terminal Amino Acid Sequence Analysis

Partial amino acid sequences for 3 of the seven major polypeptides shown in lane 4 of Figure 10 were determined.

The first 30 residues of the N-terminal sequence for the 30 kD protein are as follows:
ILE-GLY-VAL-CYS-TYR-GLY-VAL-ILE-GLY-ASN-ASN-LEU-PRO-SER-ARG-SER-ASP-VAL-VAL-GLN-LEU-TYR-ARG-SER-GLY-X-ILE-ASN-X-MET- wherein X indicates an unknown amino
acid residue.
This sequence was checked for homology with protein sequences listed in the National Cancer Institute's Supercomputer databanks. This sequence has 63% homology with the glucan endo-1,3-beta-glucosidase (EC 3.2.1.39) previously purified from barley. These results suggest that one of the mechanisms involved in the development of frost tolerance is a modification of the cell wall to increase its flexibility. The cell wall must conform to the protoplast as it shrinks during extracellular ice formation.

The first 16 amino acids of the 11 kD polypeptide are: 
AGR-SER-PHE-SER-ILE-THR-ASN-ARG-CYS-TRP-SER-PHE-THR-
VAL-PRO-GLY-
 The first 11 amino acids exhibit 55% homology with a kinase-related transforming protein (listed under the file names MUSHCK and TVMSHC). We do not yet understand the role of this protein.

The 9 kD polypeptide that exhibits antifreeze activity has been partially sequenced. The sequence representing the first twenty amino acids of the N-terminus of the polypeptide : ALA-ILE-PHE-CYS-GLY-GLN-VAL-ASN-PRO-ALA-LEU-GLY-PRO-PRO-ILE-TYR-PRO-ALA-PHE-GLY-.

### Antifreeze Activity in Different Parts of Plants

Winter rye plants grown at 5°C were separated into leaves, crowns and roots. These parts of the plants were placed in plastic bags, frozen in liquid nitrogen and allowed to thaw at room temperature. The plant tissues were then treated to obtain soluble fractions. These fractions were then assayed for their ability to modify ice crystal growth. The soluble fractions of leaves, crowns and roots all exhibited the formation of hexagonal bipyrimids in the assay for antifreeze activity. These results demonstrate that antifreeze activity is present in all parts of the vegetative plant.

### Different Species and Cultivars Having Antifreeze Activity

Sixteen different species or cultivars including both monocots and dicots (see Table 2) were grown at 5/2°C (day/night temperature) with a 16 hour daylength. Leaves from all sixteen plants were extracted by vacuum infiltration followed by centrifugation using a solution of 20 mM MgCl₂ and 10 mM ascorbic acid, pH 3.5. All sixteen intercellular extracts exhibited the ability to modify the normal pattern of ice crystal growth, although this ability varied between cultivars and species. Of the plants tested, winter rye (Secale cereale cv. Musketeer), periwinkle (Vinca minor), winter wheat (Triticum aestivum cv. Karat and Ruby) and winter barley (Hordeum vulgare cv. Acton) extracts exhibited the greatest effect on ice crystal growth with the formation of hexagonal bipyrimids, whereas winter canola (Brassica napus cv. Ceres) extract exhibited the least effect with only the formation of hexagonal discs.

### Polypeptide Having Glucanase Activity

Extracellular extracts of winter rye leaves were obtained using an extraction solution of 20 mM calcium chloride and 10 mM ascorbic acid, pH 3.5. Beta-1,3-glucanase activity was measured in extracellular extracts using the dinitrosalicylic reagent to assay the release of glucose equivalents from laminarin (II-1.0). In our experiments, the glucanase assay was optimal under the following conditions: pH 3.5, 1% laminarin, presence of CaCl₂ (as opposed to MgCl₂ or MnCl₂), 5°C, and measuring absorbance of 470 nm. In crude extracellular extracts, the beta-1,3-glucanase activity was approximately 312 mg glucose equivalents per mg total protein per hour. The extract did not exhibit beta-1,4-glucanase activity when carboxymethylcellulose was used as a substrate.

### Ice Nucleation Activity

Suspensions of single mesophyll cells were obtained from 20°C and 5°C winter rye leaves by pectolytic degradation of the leaf tissue and purification using density gradients as described in detail by Line Lapointe (1991. Photoinhibition of hardened and nonhardened rye (Secale cereale cv. Musketeer) studied with isolated thylakoids, isolated mesophyll cells, and intact leaves. Ph.D. thesis, University of Western Ontario, London, Ontario, Canada). In order to quantify the number of ice nucleators present in winter rye, dilution series of the single cell suspensions were assayed for ice nucleation activity using the droplet technique. The mean threshold ice nucleation temperature for mesophyll cells isolated from 20°C and from 5°C leaves was not significantly different and averaged -7.3°C. The mean number of mesophyll cells required to obtain an ice nucleator active at -7°C was 35,000 for 20°C leaves which can tolerate -7°C, 29,000 for 5°C/16 hour daylength leaves which can tolerate -19°C, and 11,000 for 5°C/8 hour daylength leaves which can tolerate -30°C. The results are set out in Table 3.

The composition of the ice nucleators from leaves of frost tolerant plants grown under different conditions was determined by incubating single cells in the presence of compounds and enzymes known to affect proteins, sulfhyryl groups and disulfide bonds associated with proteins, carbohydrates and phospholipids. The results are set out in Tables 4. The effect of the compound or enzyme on the ice nucleator was determined by assaying ice nucleation activity. Treating cells with 3 M urea and heating to 90°C denatures proteins and dramatically decreases ice nucleation activity. Nonspecific proteases (Pronase E and Proteinase K) also decreased ice nucleation activity. Thus the ice nucleators associated with winter rye mesophyll cells have a proteinaceous component. Reduction of disulfide bonds with dithiothreitol and reaction of free sulfhydryl groups with N-ethylmaleimide also decreased ice nucleation activity, which shows that the structure of the protein is important in producing ice nucleation activity. Boric acid and periodic acid both react with carbohydrates and both compounds reduce ice nucleation activity, thus demonstrating that the ice nucleators also contain a carbohydrate component. Finally, treatment with phospholipase C, which releases the phosphate and head group of phospholipids, also decreased ice nucleation activity. Taken, together, these results suggest that ice nucleators associated with winter rye mesophyll cells have protein, carbohydrate and phospholipid components.

### Antibodies to the Polypeptides

Polyclonal and monoclonal antibodies can be prepared for polypeptides present in extracellular extracts from cold-hardened winter rye leaves. Individual polypeptides separated by SDS-PAGE and electroeluted from gel slices are used as antigens. The antibodies are purified and used for immunopurification of the polypeptides in order to determine the ice nucleation, antifreeze and glucanase activity of each. Furthermore, the antibodies can be used for immunoassays of the polypeptides. The procedures for antibody production and purification, immunopurification of antigens and immunoassays are described in detail by Harlow and Lane (II-8.1). The detection and quantification of these polypeptides can be used in selection programs designed to decrease plant injury and yield losses caused by freezing temperatures. Previous selection programs have relied on winter survival to increase frost tolerance in overwintering crops and have been unsuccessful. Because of their sensitivity, the immunoassays provide a nondestructive means for selecting for individuals that have high concentrations of polypeptides associated with either freezing avoidance (presence of antifreeze and glucanase protein and absence of ice nucleators) or frost tolerance (presence of ice nucleation, antifreeze and glucanase protein).

### Discussion of Experimental Findings

The ice nucleation activity of the isolated polypeptides occurs in the intercellular spaces of the plant tissue. It appears that the ice nucleation proteins are bound to the cell wall and were released only by reagents that reduce disulfide bonds. This treatment also reduced ice nucleation activity.

As demonstrated by the further characterization of the polypeptides at least 7 polypeptides are synthesized at low non-freezing temperatures, namely those of Figure 9, lane 4 ranging in molecular weight from 5 kD to 36 kD and the additional two polypeptides of 60 and 68 kD found in lanes 5, 6, 7 and 8. It is also important to note the results of Figure 3 where a time course examines changes in intercellular proteins of rye leaves during cold acclimation. A co-relation exists between the degree of cold hardiness and the increased appearance of the extracellular polypeptides. The intensity of the extracellular polypeptides reaches a maximum at 78 days which corresponds to the hardiest stage of rye plants cold acclimated with a day length of 8 hours. Most of the extracellular polypeptides decrease in intensity while others were no longer detected at 102 days after germination. This result indicates the loss of freezing tolerance when plants are exposed to extended spring-like conditions. Ice nucleation activity is also indicated in Table 3 at levels as high as -7°C. This is believed to be the first report of ice nuclei of proteinaous nature in higher plants. It is also likely that several ice nucleating molecules are required in the assembly of a template upon which an ice crystal can grow. It is understood that the ice nucleating proteins are important in the extracellular spaces for the development of freezing tolerance in cold acclimated leaves.

The polypeptides as isolated and characterized in accordance with this invention establish that plants withstand frost by the combined efforts of ice nucleation, ice crystal modification by virtue of antifreeze mechanism and enzymatic alteration of the cell walls to allow the cell walls to increase flexibility during development of ice crystals in the intercellular spaces. It has been demonstrated that cold acclimated winter rye leaves are not injured by ice formation even when the leaves are first undercooled to temperatures as low as -12°C. Non-acclimated winter rye leaves exhibit injury whenever ice forms. Hence, in the development of frost tolerance a gradual acclimation is required. In accordance with this invention, ice formation in the extracellular spaces indicates that it is not the presence of antifreeze proteins which determines the lowest limit of cell survival at freezing temperatures. As temperatures decrease intracellular water is lost to the growing extracellular ice masses and the cells themselves become dehydrated. The lowest temperature which frost tolerant plants survive is therefore correlated with desiccation tolerance of the cells (II-16, 18).

Conventional breeding programs have failed to improve frost resistance in crop plants because physiological markers specific for frost tolerance are not yet available (II-2). The discovery of ice nucleation and antifreeze proteins intrinsically produced by a frost tolerant plant as demonstrated by this invention represents an important breakthrough in agriculture for two reasons. First of all, the ice nucleation and antifreeze polypeptides are the first polypeptides demonstrated to be directly involved in the process of freezing tolerance in plants. Antifreeze and ice nucleation polypeptides may prove useful as selection markers for increasing frost tolerance in overwintering crops. Secondly, further isolation and characterization of the ice nucleation and antifreeze protein will be useful for increasing survival and productivity. In the future, it may be possible to raise crops successfully in regions or in seasons where crop production is now limited by freezing temperatures.

As already indicated the polypeptides are useful in production of frozen foods and cryogenic storage of biological tissues. Treatment of frozen foods with the polypeptides can ensure superior food quality upon thawing of the product. Also, with the manufacture of products such as ice cream as well as in the cryopreservation of biological tissues it is desirable to have a minute crystalline structure. The use of the antifreeze proteins in limiting crystalline size and in preventing recrystallization would be very useful in providing a superior product.

Although preferred embodiments of the invention are described herein in detail, it will be understood by those skilled in the art that variations may be made thereto without departing from the spirit of the invention or the scope of the appended claims.

**TABLE 1**

| Amino Acid Compositions (mol%) of Major Winter Rye Polypeptides Found in Column Fractions Exhibiting Antifreeze Activity (peak 2). | | | | | | |
|---|---|---|---|---|---|---|
| polypeptides (kD) | | | | | | |
| Amino acid residue | 36 | 30 | 24 | 22 | 11 | 9 |
| ASX | 11.1 | 9.2 | 13.0 | 11.4 | 11.8 | 15.5 |
| GLX | 8.1 | 6.8 | 7.8 | 7.9 | 11.6 | 6.7 |
| HYP | 0 | 0 | 0 | 0 | 0 | 0 |
| SER | 1.0 | 8.7 | 7.5 | 7.5 | 8.9 | 9.2 |
| GLY | 16.8 | 12.8 | 14.5 | 16.8 | 18.3 | 17.9 |
| HIS | 0 | 0.4 | 0.7 | 0.5 | 0 | 0.9 |
| ARG | 3.7 | 4.2 | 5.2 | 4.0 | 3.0 | 4.7 |
| THR | 5.5 | 5.6 | 9.3 | 7.9 | 5.2 | 7.1 |
| ALA | 8.8 | 15.2 | 12.0 | 9.0 | 12.2 | 7.9 |
| PRO | 6.0 | 7.5 | 5.6 | 5.2 | 6.0 | 4.3 |
| TYR | 5.5 | 5.3 | 5.6 | 5.2 | 6.0 | 4.3 |
| VAL | 5.3 | 6.7 | 5.2 | 6.0 | 4.6 | 6.0 |
| MET | 1.2 | 1.3 | 0.9 | 0.7 | 0.8 | 0.4 |
| CYS | 5.1 | 0.9 | 1.2 | 2.2 | 2.0 | - |
| ILU | 2.8 | 4.6 | 3.0 | 2.6 | 2.7 | 3.3 |
| LEU | 4.8 | 6.9 | 4.4 | 4.5 | 3.8 | 5.6 |
| PHE | 3.2 | 3.1 | 3.1 | 3.9 | 2.8 | 3.3 |
| LYS | 2.1 | 1.3 | 1.1 | 1.9 | 1.6 | 1.6 |
| Winter rye polypeptides were separated by SDS-PAGE and blotted onto PVDF. For cysteine, blots were wetted with methanol, oxidized with fresh performic acid for 2.5 h at 5°C and dried under vacuum. Blots were hydrolyzed in 6 NHCL with 1 % phenol for 24 h at 110°C. Amino acids were derivatized with phenylisothiocyanate and analyzed using the Waters PICO-TAG System by the HSC/Pharmacia Biotechnology Service Center, Toronto, Ontario, Canada. | | | | | | |

**Table 2.**

| Antifreeze activity of four species of monocotyledonous plants and two species of dicotyledonous plants. Protein concentration and relative antifreeze activity of extracellular extracts of leaves from plants grown at 5°C with a 16 hour day. | | | |
|---|---|---|---|
| Species | Cultivar | Protein concentration (mg/g fresh weight) | Rank of antifreeze activity¹ |
| **Monocots:** | | | |
| *Secale cereale* (winter rye) | | | |
| | Musketeer | 0.15 | 5 |
| *Triticum aestivum* (wheat) | | | |
| Soft-white winter | | | |
| | Annette | 0.30 | 3 |
| | Augusta | 0.22 | 2 |
| | Frankenmuth | 0.09 | 2 |
| | Frederick | 0.16 | 4 |
| | Rebecca | 0.04 | 2 |
| Hard-red winter | | | |
| | Absolvent | 0.15 | 4 |
| | Karat | 0.23 | 5 |
| | Ruby | 0.24 | 5 |
| Spring | | | |
| | Katepwa | 0.12 | 4 |
| *Hordeum vulgare* (winter barley) | | | |
| | Acton | 0.05 | 5 |
| | **Elmira** | 0.25 | 3 |
| | **Halton** | 0.25 | 3 |
| | Huron | 0.11 | 1 |
| *Avena sativa* (winter oats) | | | |
| | Ogle | 0.04 | 4 |

| **Dicots:** | | | |
|---|---|---|---|
| *Brassica napus* (winter canola) | | | |
| | Ceres | 0.04 | 1 |
| *Vinca minor* (periwinkle) | | 0.04 | 5 |

| | | | |
|---|---|---|---|
| ¹Ranks represent increasing antifreeze activity based on ice crystal morphology as follows: (1) hexagonal disc, (2) short hexagonal column, (3) long hexagonal column, (4) partial hexagonal bipyramid and (5) complete hexagonal bipyrimid. | | | |

**TABLE 3**

| Comparison Between Hardened and Nonhardened Rye Leaves in the Number of Mesophyll Cells Required to Obtain an Active Ice Nucleator | | | |
|---|---|---|---|
| Growth Conditions | Number of Replicates | Mean Threshold Nucleation Temperature (Mean ± S.D.) | Mean Number of Cells Per Nucleator (Mean ± S.D.) |
| Nonhardened Rye (20°C/16 h day) | 12 | -7.2 ± 0.6 | 35,266 ± 25,977 |
| Hardened Rye (5°C/16 h day) | 14 | -7.1 ± 0.8 | 28,511 ± 39,563 |
| Hardened Rye (5°C/8 h day) | 16 | -7.6 ± 0.6 | 10,847 ± 11,211 |

**TABLE 4**

| CHARACTERIZATION OF ICE NUCLEATORS ASSOCIATED WITH WINTER RYE MESOPHYLL CELLS | | | | |
|---|---|---|---|---|
| | | GROWTH CONDITIONS | | |
| TREATMENT | INDICATION | 20°C.16hr | 5°C.16hr | 5°C.8hr |
| | | *mean threshold ice nucleation temperature = S.D.(n*) | | |
| Crude | | -7.2 ± 0.5(15) | -7.2 ± 0.7(17) | -7.4 ± 0.7(19) |
| Boric acid (4mM) | Carbohydrates | -12.1 ± 0.6(3)^{a} | -11.0 ± 0.5(3)^{a} | -10.6 ± 1.1(3)^{a} |
| Periodic acid (2mM) | Carbohydrates | -11.4 ± 1.0(3)^{a} | -10.7 ± 1.5(3)^{a} | -9.4 ± 1.8(5)^{a} |
| Phospholipase C (3mg/ml) | Lipids | -10.8 ± 1.5(3)^{a} | -8.7 ± 1.1(3)^{b} | -9.5 ± 0.4(7)^{a} |
| Heat (90°C 10 minutes) | Proteins | -10.7 ± 1.6(5)^{a} | -12.0 ± 0.5(3)^{a} | -11.5 = 1.8(5)^{a} |
| Urea (3M) | Proteins | -11.2 ± 1.6(4)^{a} | -11.4 ± 1.2(5)^{a} | -13.4 ± 0.1(3)^{a} |
| Pronase E (3mg/mL) | Proteins | -11.1 ± 2.6(3)^{a} | -9.3 ± 0.8(3)^{a} | -9.1 ± 0.5(7)^{a} |
| Proteinase K (3mg/mL) | Proteins | -7.5 ± 0.4(3)^{c} | -10.2 ± 0.1(3)^{a} | -10.9 ± 0.7(3)^{a} |
| N-ethylmaleimide (1mM) | .SH groups | -12.1 ± 1.9(5)^{a} | -11.1 ± 0.6(3)^{a} | -12.1 ± 3.3(3)^{a} |
| Dithiothreitol (50mM) | Disulphides | -9.2 ± 0.7(4)^{a} | -10.1 ± 1.0(5)^{a} | -10.4 ± 1.3(5)^{a} |

| | | | | |
|---|---|---|---|---|
| ^{a} - test of significance at 99.9% | | | | |
| ^{b} - test of significance at 95% | | | | |
| ^{c} - not significantly different from crude | | | | |

## Claims

1. An in vitro method for limiting crystallization or recrystalization of ice and/or modifying ice growth or limiting frost, ice or cold damage in biological tissue or frozen food product or a solution, said method comprising applying an effective amount of an antifreeze polypeptide having the properties of:
i) being a polypeptide produced in extracellular spaces between cells of a cold acclimated frost tolerant plant, and
ii) having antifreeze or ice nucleation promoting properties.

2. A method as claimed in Claim 1, wherein the polypeptide is one which is developed in a frost tolerant Secale cereale leaf and is selected from the group consisting of antifreeze polypeptides having the apparent molecular weights as determined by SDS-PAGE of about of about 5 kD, about 9 kD, about 11 kD, about 22 kD, about 24 kD, about 30 kD and about 36 kD.

3. A method as claimed in Claim 1, wherein said polypeptide is one which is developed in a frost tolerant plant selected from the group consisting of Secale cereale, Triticum aestivum, Hordeum vulgare, Avena sativa, Brassica napus, and Vinca minor.

4. A method as claimed in Claim 1, wherein said plant comprises a vegetative plant.

5. A method as claimed in any preceding claim, wherein said polypeptide is applied in an extract from said frost tolerant plant.

6. A method as claimed in Claim 5, wherein said extract comprises an extracellular extract.

7. A frozen food preparation comprising a polypeptide **characterised by** having the properties of:
i) being a polypeptide produced in extracellular spaces between cells of a cold acclimated frost tolerant plant, and
ii) having antifreeze or ice nucleation promoting properties.

8. A frozen food preparation as claimed in Claim 7, wherein the polypeptide is one which is developed in a frost tolerant Secale cereale leaf and is selected from the group consisting of antifreeze polypeptides having the apparent molecular weights as determined by SDS-PAGE of about of about 5 kD, about 9 kD, about 11 kD, about 22 kD, about 24 kD, about 30 kD and about 36 kD.

9. A frozen food preparation as claimed in Claim 7, wherein said polypeptide having the properties of being developed in a vegetative portion of a frost tolerant plant is selected from the group consisting of Secale cereale, Triticum aestivum, Hordeum vulgare, Avena sativa, Brassica napus, and Vinca minor.

10. The frozen food preparation as claimed in Claim 9, wherein said frozen food preparation comprises ice cream or fruit.

11. A method for determining whether a plant extract has antifreeze activity, comprising a) obtaining an extract from a vegetative portion of a plant grown at low temperature and b) determining the ability of the extract to modify ice growth, inhibit recrystallization or inhibit thermal hysteresis.

12. An isolated polypeptide **characterised by** having the properties of:
i) being a polypeptide produced in extracellular spaces between cells of a cold acclimated frost tolerant plant, and
ii) having antifreeze or ice nucleation promoting properties.

13. An antibody which is specifically reactive with a polypeptide as claimed in Claim 12.

14. The use of antibodies as claimed in claim 14, in an immunoassay to determine whether a plant contains antifreeze or ice nucleation controlling polypeptides.

## Patentansprüche

1. *In-vitro*-Verfahren zur Beschränkung der Kristallisation oder Rekristallisation von Eis und/oder zur Modifizierung des Wachstums von Eis oder zur Beschränkung von Schäden durch Frost, Eis oder Kälte in biologischem Gewebe, einem gefrorenen Lebensmittel oder einer Lösung, wobei das Verfahren das Aufbringen bzw. Anwenden einer effektiven Menge eines Frostschutzpolypeptids umfasst, das folgende Eigenschaften hat:
i) es handelt sich um ein Polypeptid, das in extrazellulären Räumen zwischen den Zellen einer an Kälte akklimatisierten frostresistenten Pflanze erzeugt wird, und
ii) es hat Frostschutzeigenschaften bzw. Eigenschaften, die Bildung von Eiskristallisationskeimen beschleunigen.

2. Verfahren nach Anspruch 1, bei dem das Polypeptid eines ist, das sich in einem frostresistenten Blatt von *Secale cereale* entwickelt und ausgewählt wird aus der Gruppe, bestehend aus Frostschutzpolypeptiden mit durch SDS-PAGE bestimmten scheinbaren Molekulargewichten von etwa 5 kD, etwa 9 kD, etwa 11 kD, etwa 22 kD, etwa 24 kD, etwa 30 kD und etwa 36 kD.

3. Verfahren nach Anspruch 1, bei dem das Polypeptid eines ist, das sich in einer frostresistenten Pflanze entwickelt, ausgewählt aus der aus *Secale cereale, Triticum aesrivum, Hordeum vulgare, Avena sativa, Brassica napus* und *Vinca minor* bestehenden Gruppe.

4. Verfahren nach Anspruch 1, bei dem die Pflanze eine vegetative Pflanze umfasst.

5. Verfahren nach einem der vorstehenden Ansprüche, bei dem das Polypeptid in einem Extrakt aus der frostresistenten Pflanze aufgebracht bzw. angewandet wird.

6. Verfahren nach Anspruch 5, bei dem der Extrakt einen extrazellulären Extrakt umfasst.

7. Gefrorene Lebensmittelzubereitung, umfassend ein Polypeptid, das durch folgende Eigenschaften **gekennzeichnet** ist:
i) es handelt sich um ein Polypeptid, das in extrazellulären Räumen zwischen den Zellen einer an Kälte akklimatisierten frostresistenten Pflanze erzeugt wird, und
ii) es hat Frostschutzeigenschaften bzw. Eigenschaften, die Bildung von Eiskristallisationskeimen beschleunigen.

8. Gefrorene Lebensmittelzubereitung nach Anspruch 7, in der das Polypeptid eines ist, das sich in einem frostresistenten Blatt von *Secale cereale* entwickelt und ausgewählt wird aus der Gruppe, bestehend aus Frostschutzpolypeptiden mit durch SDS-PAGE bestimmten scheinbaren Molekulargewichten von etwa 5 kD, etwa 9 kD, etwa 11 kD, etwa 22 kD, etwa 24 kD, etwa 30 kD und etwa 36 kD.

9. Gefrorene Lebensmittelzubereitung nach Anspruch 7, in der das Polypeptid die Eigenschaft hat, dass es sich im vegetativen Teil einer frostresistenten Pflanze entwickelt, ausgewählt aus der aus *Secale cereale, Triticum aestivum, Hordeum vulgare, Avena sativa, Brassica napus* und *Vinca minor* bestehenden Gruppe.

10. Gefrorene Lebensmittelzubereitung nach Anspruch 9, wobei die gefrorene Lebensmittelzubereitung Eiscreme oder Obst umfasst.

11. Verfahren zur Bestimmung, ob ein Pflanzenextrakt eine Frostschutzwirkung hat, umfassend a) den Erhalt eines Extrakts aus einem vegetativen Teil einer bei niedriger Temperatur gezüchteten Pflanze und b) die Bestimmung der Fähigkeit des Extrakts, das Eiswachstum zu modifizieren, die Rekristallisation zu inhibieren oder die thermische Hysterese zu inhibieren.

12. Isoliertes Polypeptid, das durch folgende Eigenschaften **gekennzeichnet** ist:
i) es handelt sich um ein Polypeptid, das in extrazellulären Räumen zwischen den Zellen einer an Kälte akklimatisierten frostresistenten Pflanze erzeugt wird, und
ii) es hat Frostschutzeigenschaften bzw. Eigenschaften, die Bildung von Eiskristallisationskeimen beschleunigen.

13. Antikörper, der spezifisch mit einem Polypeptid gemäß Anspruch 12 reagiert.

14. Verwendung von Antikörpern nach Anspruch 14 in einem Immunoassay, um zu bestimmen, ob eine Pflanze Polypeptide enthält, die als Frostschutz wirken bzw. die Bildung von Eiskristallisationskeimen steuern.

## Revendications

1. Procédé *in vitro* de limitation de la cristallisation ou de la recristallisation de la glace et/ou de modification du développement de la glace ou de limitation des dommages provoqués par le gel, la glace ou le froid dans un tissu biologique, un produit alimentaire surgelé ou une solution, ledit procédé comprenant l'exposition à une quantité efficace d'un polypeptide antigel ayant les propriétés :
i) d'être un polypeptide produit dans les espaces extracellulaires entre les cellules d'une plante adaptée au froid et tolérante au gel, et
ii) d'avoir des propriétés antigel ou de stimulation de la nucléation de la glace.

2. Procédé selon la revendication 1, dans lequel le polypeptide est un polypeptide se formant dans une feuille de *Secale cereale* tolérante au gel et choisi dans le groupe comprenant des polypeptides antigel d'un poids moléculaire apparent, comme déterminé par SDS-PAGE, d'environ 5 kD, d'environ 9 kD, d'environ 11 kD, d'environ 22 kD, d'environ 24 kD, d'environ 30 kD et d'environ 36 kD.

3. Procédé selon la revendication 1, dans lequel ledit polypeptide est un polypeptide se formant dans une plante tolérante au gel choisie dans le groupe comprenant *Secale cereale, Triticum aestivum, Hordeum vulgare, Avena sativa, Brassica napus* et *Vinca minor.*

4. Procédé selon la revendication 1, dans lequel ladite plante comprend une plante végétative.

5. Procédé selon l'une quelconque des revendications précédentes, dans lequel ledit polypeptide est appliqué sous la forme d'un extrait obtenu à partir de ladite plante tolérante au gel.

6. Procédé selon la revendication 5, dans lequel ledit extrait comprend un extrait extracellulaire.

7. Préparation alimentaire surgelée comprenant un polypeptide **caractérisé par** les propriétés suivantes :
i) être un polypeptide produit dans les espaces extracellulaires entre les cellules d'une plante adaptée au froid et tolérante au gel, et
ii) posséder des propriétés antigel ou de stimulation de la nucléation de la glace.

8. Préparation alimentaire surgelée selon la revendication 7, dans laquelle le polypeptide est un polypeptide se formant dans une feuille de *Secale cereale* tolérante au gel, et choisi dans le groupe comprenant des polypeptides antigel d'un poids moléculaire apparent, comme déterminé par SDS-PAGE, d'environ 5 kD, d'environ 9 kD, d'environ 11 kD, d'environ 22 kD, d'environ 24 kD, d'environ 30 kD et d'environ 36 kD.

9. Préparation alimentaire surgelée selon la revendication 7, dans laquelle ledit polypeptide a comme propriété de se former dans la portion végétative d'une plante tolérante au gel choisie dans le groupe comprenant *Secale cereale, Triticum aestivum, Hordeum vulgare, Avena sativa, Brassica napus* et *Vinca minor.*

10. Préparation alimentaire surgelée selon la revendication 9, dans laquelle ladite préparation alimentaire surgelée comprend crème glacée ou fruits.

11. Procédé de mise en évidence d'une éventuelle activité antigel d'un extrait de plante, comprenant a) l'obtention d'un extrait à partir d'une portion végétative d'une plante cultivée sous basse température et b) la détermination de la capacité de l'extrait à modifier le développement de la glace, à inhiber la recristallisation ou à inhiber l'hystérésis thermique.

12. Polypeptide isolé **caractérisé par** les propriétés suivantes :
i) être un polypeptide produit dans les espaces extracellulaires entre les cellules d'une plante adaptée au froid et tolérante au gel, et
ii) posséder des propriétés antigel ou de stimulation de la nucléation de la glace.

13. Anticorps particulièrement réactif à l'égard d'un polypeptide selon la revendication 12.

14. Utilisation d'anticorps selon la revendication 13, dans le cadre d'un essai immunologique visant à déterminer si une plante contient des polypeptides antigel ou contrôlant la nucléation de la glace.
